# FASCICULE DE BREVET EUROPEEN

(11) **EP 3 651 730 B1**
(45) Date de publication et mention de la délivrance du brevet: **07.06.2023**
(21) Numéro de dépôt: 18752576.1
(22) Date de dépôt: 10.07.2018
(51) Int. Cl.: A61K 8/34, A61K 8/44, A61Q 5/06, A61K 8/65, A61K 8/92

(54) **NOUVELLE COMPOSITION POUR LE LISSAGE DES CHEVEUX**
NEUE ZUSAMMENSETZUNG ZUM GLÄTTEN VON HAAREN
NEW COMPOSITION FOR STRAIGHTENING HAIR

(30) Priorité: 11.07.2017 FR 1756543; 24.11.2017 FR 1761139
(43) Date de publication de la demande: 20.05.2020
(73) Titulaire: Di Visco, 69230 Saint Genis Laval (FR)
(72) Inventeur: VISCOGLIOSI, Sébastien Frédéric, 69008 Lyon (FR); GARCIA, Isabel, 69008 Lyon (FR); GUERIN, Didier, 31540 Saint Félix Lauragais (FR)
(74) Mandataire: Germain Maureau
(86) Numéro de dépôt international: PCT/FR2018/051730
(87) Numéro de publication internationale: WO 2019/012219

(56) Documents cités:
- FR-A1- 3 030 241
- US-A1- 2009 111 750
- US-B1- 9 381 144

## Description

La présente invention a pour objet une nouvelle composition permettant le lissage des cheveux comprenant un acide aminé, une protéine, un alcool et une huile végétale, ainsi qu'un procédé de lissage des cheveux mettant en oeuvre ladite composition.

Dans le domaine capillaire, le lissage de cheveux est une technique de coiffure qui permet de défriser les cheveux de façon temporaire ou sur une plus longue durée.

Les consommateurs souhaitant lisser leurs cheveux cherchent non seulement à obtenir une chevelure lisse présentant une bonne tenue mais également à obtenir un résultat qui soit durable dans le temps en résistant notamment aux shampooings subséquents.

Pour modifier la forme de la chevelure, de façon durable, on utilise généralement des traitements thermiques. Ces traitements permettent une modification visuelle de l'aspect de la coiffure, tant en termes de frisure ou de frizzotis qu'en termes de volume global de la chevelure. De tels traitements permettent d'aboutir à un aspect visuel plus lisse, un gain substantiel de brillance, une résistance à l'humidité et à la chaleur pour un maintien de la coiffure toute la journée.

Ces traitements thermiques sont généralement complétés (ou précédés) de traitements « chimiques » de la chevelure par des compositions particulières.

Un premier traitement complémentaire repose sur l'utilisation de compositions à base de réducteurs thiolés. Ces technologies nécessitent un respect rigoureux des conditions d'application préconisées par les fournisseurs, notamment en termes de quantité et de temps de pause. En outre, elles peuvent être contre-indiquées sur des cheveux trop sensibilisés et ne pas être compatibles avec l'application le même jour d'autres traitements tels que les colorations ou les décolorations. Elles sont par ailleurs malodorantes.

Un second traitement complémentaire repose sur l'utilisation de compositions à base de formol (ou formaldéhyde) et de ses dérivés. Ces traitements ont pour particularité d'être robustes, parfaitement compatibles avec tous les autres traitements capillaires classiques, tels que les lissages thiolés mentionnés précédemment, les défrisages alcalins, les colorations ou les décolorations de tous types, effectués avant ou après. Ils confèrent aux cheveux une excellente discipline, une très forte brillance et une facilité de soin au quotidien. Cependant, ces produits nécessitent l'utilisation de fer à lisser à une température de 180-230°C ce qui endommage très fortement les cheveux à court ou moyen terme. En outre, ces méthodes de lissage sont aussi très longues à appliquer car il faut travailler mèche par mèche et le temps global moyen pour lisser une chevelure de type « afro-caribéen » est de 4 à 9 heures. Enfin, pour des raisons toxicologiques, l'utilisation de certains de ces composés est maintenant interdite et/ou réglementée.

De nouveaux traitements complémentaires ont ainsi été développés en vue de faciliter le procédé de lissage et d'éviter l'utilisation de ces substances toxiques et nocives pour la santé des utilisateurs.

Ainsi, la demande de brevet internationale WO 2011/104282 décrit l'utilisation de compositions à base d'acides, en particulier l'acide glyoxylique. Cette demande décrit en particulier un procédé pour lisser de manière semipermanente les cheveux, consistant à appliquer une solution d'alphacétoacide sur les cheveux pendant 15 à 120 minutes, puis à sécher et enfin à lisser au fer, à une température d'environ 200°C, la chevelure. L'alphacétoacide employé est de préférence l'acide glyoxylique.

La demande de brevet français FR 3030241 décrit quant à elle un procédé de lissage des cheveux par application d'une composition comprenant un acide aminé et un polyol suivie d'une étape de lissage au moyen d'un fer de lissage à une température d'au moins 100°C.

Toutefois, on a constaté que l'acide glyoxylique peut ne pas être bien toléré, en particulier quand le cuir chevelu est sensible et/ou irrité. Sa volatilité, amplifiée par l'utilisation de chaleur du fer, pose également problème.

En outre, l'ensemble des traitements complémentaires récemment développés sont finalement inefficaces ou, à tout le moins, d'une efficacité limitée, et nécessitent tous l'utilisation d'un fer à lisser à haute température, ce qui détériorent les cheveux en provoquant notamment leur casse.

Ainsi, à la date de la présente invention, il demeure nécessaire de mettre au point une composition permettant le lissage des cheveux de manière efficace et durable dans des conditions d'application aisée sans détériorer la qualité les cheveux.

Or, il a maintenant été trouvé une composition permettant un lissage des cheveux efficace et durable, selon un procédé d'application aisé à mettre en oeuvre tout en préservant la qualité des cheveux.

La présente invention a donc pour objet une composition pour le lissage des cheveux comprenant :
- un acide aminé protéinogène ;
- une protéine ;
- un alcool ; et
- une huile végétale.

La composition selon la présente invention permet un lissage efficace et durable des cheveux, selon un procédé d'application plus aisé et préservant la qualité des cheveux, notamment du fait de l'absence d'utilisation de fer à lisser.

Dans le cadre de la présente invention :
- on entend par « lissage » tout traitement permettant d'aplatir ou défriser le cheveu afin de lui donner une surface apparente lisse;
- on entend par « acide aminé » tout acide aminé connu de l'homme du métier, en particulier les acides aminés de formule générale (I) suivante 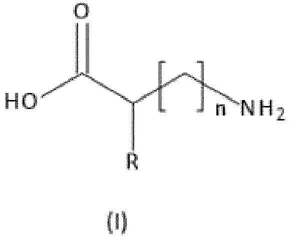 dans laquelle
   - R représente un atome d'hydrogène, un groupe C₁-C₅-alkyle éventuellement substitué par au moins un groupement choisi parmi hydroxyle, amino ; - C(O)-OH ; -S(O)₂-OH ; -C(O)-O⁻, M+ ; -S(O)₂-O⁻, M+ ;
   - M+ représentant un contre ion cationique tel que métal alcalin, alcalinoterreux, ou ammonium ;
   - n vaut 0 ou 1 ;
   ainsi que leurs formes bétaïnes ; leurs isomères optiques ; leurs solvates tels que les hydrates, et leurs sels, d'acide ou de base, organiques ou minéraux ;
- on entend par « huile végétale » tout corps gras extrait d'une plante oléagineuse, c'est-à-dire une plante dont les graines, noix ou fruits contiennent des lipides ;
- on entend par « protéine » toute protéine, hétéroprotéine ou dérivé protéique tel qu'une protéine greffée sur une hétéromolécule pouvant être du silicone, un composé glucydique, un acide gras ou dérivé, un tensio-actif de caractère anionique, cationique, non-ionique ou amphotère, de taille et de forme variable, formée d'une ou plusieurs chaîne(s) chargée(s) en acides aminés et reliée(s) par des liaisons peptidiques. De préférence, la protéine est de structure tertiaire ou quaternaire ;
- on entend par « Cₓ-C_{y}-alkyle », une chaîne hydrocarbonée saturée, linéaire ou ramifiée, et comportant de x à y atomes de carbone ;
- on entend par « alcool » toute chaîne carbonée linéaire ou ramifiée contenant de 1 à 10 atomes de carbone substituée par un groupement hydroxyle, un aminoalcool, un aldol ou un cétol. De préférence, « alcool » désigne tout composé linéaire ou ramifié de formue CₙH₂ₙ₊₁OH dans laquelle n est un entier compris entre 1 et 10 ; et
- on entend par « polyol » tout groupe C₁-C₁₀-alkyle substitué par au moins deux groupes hydroxyle. De préférence, on entend par « polyol » tout composé choisi parmi la glycérine, le propylène glycol, le propane-1,3-diol ou le sorbitol.

De plus, dans le cadre de la présente invention, et sauf mention contraire, les proportions exprimées en % correspondent à des pourcentages massiques par rapport au poids total de l'entité considérée.

La composition selon la présente invention contient donc un acide aminé protéinogène, une protéine, un alcool et une huile végétale. De préférence, la présente invention a pour objet une composition telle que décrite précédemment présentant les caractéristiques suivantes, prises seules ou en combinaison :
- l'acide aminé protéinogène est choisi parmi l'acide aspartique, l'acide glutamique, l'asparagine, la carnitine, la cystéine, la glutamine, l'histidine, la leucine, l'isoleucine, la méthionine, la N-phenylalanine, la proline, l'hydroxyproline, la threonine, le tryptophan, la tyrosine, la valine, la glycine, l'alanine, la sérine, la béta alanine, la taurine, la lysine et l'arginine ou l'un de leurs sels, d'acide ou de base, organiques ou minéraux. De façon tout à fait préférée, l'acide aminé protéinogène est choisi parmi l'acide glutamique, la cystéine et l'arginine;
- la protéine est choisie parmi la créatine éventuellement hydrolysée, la kératine éventuellement hydrolysée, la protéine de blé éventuellement hydrolysée, la protéine de soie éventuellement hydrolysée, la protéine de soja éventuellement hydrolysée, le collagène éventuellement hydrolysé, l'élastine éventuellement hydrolysée, la protéine de pois éventuellement hydrolysée, la protéine de riz éventuellement hydrolysée, la protéine de conchioline éventuellement hydrolysée et la protéine de lactosérum éventuellement hydrolysée. De façon tout à fait préférée, la protéine est choisie parmi la créatine, la kératine, la protéine de blé éventuellement hydrolysée, la protéine de soie éventuellement hydrolysée et la protéine de soja éventuellement hydrolysée.

- l'alcool est choisi parmi le butanol, l'isopropanol et l'éthanol. De préférence encore, l'alcool est choisi comme étant l'éthanol ;
- l'huile végétale contient au moins 1% de soufre ou d'acides aminés soufrés. De préférence encore, l'huile végétale est choisie parmi l'huile d'amande douce, l'huile d'olive, l'huile d'argan, l'huile d'Haarlem, l'huile de Babassu, l'huile de brocoli, l'huile d'Avocat, l'huile de Lin, l'huile d'abricot, l'huile de concombre, l'huile de nigelle, l'huile de noix du brésil, l'huile de pépins de raisin, l'oléine de karité, l'huile de sésame et l'huile de tomate. De façon tout à fait préférée, l'huile végétale est choisie comme étant l'huile de brocoli ou l'huile d'Haarlem ;
- la composition contient en outre un polyol ; et/ou
- la composition contient également des pigments ou colorants permettant d'obtenir une coloration des cheveux.

La composition selon l'invention peut se présenter sous la forme d'une composition unique ou sous la forme d'un kit comprenant deux, trois ou quatre compositions séparées contenant chacune une ou plusieurs des ingrédients de la composition selon la présente invention. De manière préférée, la présente invention a également pour objet un kit utile pour le lissage des cheveux comprenant :
- d'une part une composition contenant l'acide aminé protéinogène tel que défini précédemment et la protéine telle que définie précédemment ; et
- d'autre part une composition contenant l'alcool tel que défini précédemment et l'huile végétale telle que définie précédemment.

De préférence, la présente invention a pour objet un kit tel que décrit précédemment présentant les caractéristiques suivantes, prises seules ou en combinaison :
- le kit contient de 50% à 70% de la composition comprenant l'acide aminé protéinogène et la protéine et de 30% à 50% de la composition comprenant l'alcool et l'huile végétale ;
- la composition contenant l'acide aminé protéinogène et la protéine contient de 0,1% à 20% d'acide aminé tel que défini précédemment, de préférence de 0,1% à 5% d'acide aminé protéinogène tel que défini précédemment, de préférence encore de 0,1% à 1% d'acide aminé protéinogène tel que défini précédemment ;
- la composition contenant l'acide aminé protéinogène et la protéine contient de 0,1% à 20% de protéine telle que définie précédemment, de préférence encore de 0,1% à 5% de protéine telle que définie précédemment, de façon tout à fait préférée de 0,1% à 2% de protéine telle que définie précédemment;
- la composition contenant l'acide aminé protéinogène et la protéine se présente sous la forme de crème, de gel, d'émulsion huile dans eau ou eau dans huile, de lotion capillaire ou de shampooing. De préférence encore la composition contenant l'acide aminé protéinogène et la protéine se présente sous la forme d'une crème ;
- la composition contenant l'alcool et l'huile végétale se présente sous la forme de crème, de gel, d'émulsion huile dans eau ou eau dans huile, de lotion bi-phasique ou de shampooing. De préférence encore la composition contenant l'alcool et l'huile végétale se présente sous la forme d'une lotion bi-phasique ;
- la composition contenant l'alcool et l'huile végétale contient de 1% à 50% d'alcool tel que défini précédemment, de préférence encore de 10% à 50% d'alcool tel que défini précédemment, de façon tout à fait préférée de 10% à 30% d'alcool tel que défini précédemment ;
- la composition contenant l'alcool et l'huile végétale contient de 50% à 95% d'huile végétale telle que définie précédemment, de préférence encore de 50% à 80% d'huile végétale telle que définie précédemment, de façon tout à fait préférée de 70% à 80% d'huile végétale telle que définie précédemment;
- la composition contenant l'alcool et l'huile végétale contient en outre un polyol ; et/ou
- les pigments ou colorants pour la coloration capillaire sont intégrés dans la préparation contenant les acides aminés et les protéines ou dans une préparation complémentaire.

La composition selon la présente invention, qu'elle se présente sous forme de kit ou non, peut-être appliquée selon tout procédé connu de l'homme du métier. De préférence, la composition selon la présente invention est apppliquée selon le procédé comprenant les étapes suivantes :
- lorsque la composition se présente sous forme de kit tel que décrit précédemment, mélanger la composition comprenant l'acide aminé protéinogène et la protéine telle que définie précédemment et la composition comprenant l'alcool et l'huile végétale telle que définie précédemment en vue d'obtenir la composition selon la présente invention ;
- appliquer le produit obtenu (i.e. la composition selon la présente invention) mèches par mèches de la racine à la pointe
- laisser agir le produit pendant une durée pouvant varier de 15 minutes à 45 minutes ;
- sécher les cheveux, de préférence à l'air chaud par exemple à l'aide d'un séchoir ;
- rincer les cheveux à l'eau ; et
- procéder au coiffage avec un séchoir.

Le procédé selon la présente invention peut être aisément mis en oeuvre et permet un lissage des cheveux efficace et durable sans utilisation de fer à lisser, préservant ainsi la qualité des cheveux.

Le procédé selon la présente invention peut en outre être complété par :
- l'application d'un shampooing préalablement à l'application de la composition selon l'invention ;
- un pré-sèchage partiel des cheveux avant l'application de la composition selon l'invention ; et/ou
- l'application d'une composition type « masque » capillaire, hydrophobe ou non, après le rinçage à l'eau et avant le coiffage des cheveux.

La présente invention est illustrée de manière non limitative par les exemples suivants.

### Exemple 1 - Kit selon l'invention

Un kit contenant :
- une composition de lissage se présentant sous la forme d'une crème comprenant un acide aminé protéinogène et une protéine ;
- et un activateur biphasique se présentant sous la forme d'une lotion biphasique comprenant un alcool et une huile végétale ;
dont les compositions sont rapportées respectivement dans les Tableaux 1 et 2 suivants est préparé.

**Tableau 1 - Composition de lissage**

| **Ingrédients** | | **Quantité (% p/p)** |
|---|---|---|
| Acide glutamique | | 0.1 |
| Cystéine HCl | | 0.3 |
| Arginine | | 0.1 |
| Créatine | | 0.05 |
| Protéine de blé hydrolysée | | 0.3 |
| Protéine de soja hydrolysée | | 0.3 |
| Kératine hydrolysée | | 0.3 |
| Polyquaternium 7 | | 0.25 |
| Hydroxypropyl starch phosphate | | 0.25 |
| Parfum | | 0.3 |
| Phénoxyéthanol | | 0.3 |
| Amisol Trio^{™} | | 0.2 |
| | *Phospholipides* | |
| | *Huile de soja glycine* | |
| | *Glycolipides* | |
| | *Stérol de soja glycine* | |
| Acide ascorbique | | 0.05 |
| Alcool cétéarylique | | 4 |
| Chlorure de cétrimonium | | 2 |
| Ceteareth20 | | 0.3 |
| Glycérine | | 0.25 |
| Ethylhexylpalmitate | | 0.25 |
| *Triticum vulgare* (huile de germe de blé) | | 0.25 |
| *Orbignya oleifera* (huile de graines de Babassu) | | 0.25 |
| Acide lactique | | 0.05 |
| Acide citrique | | 0.05 |
| Ceramide-3 | | 0.05 |
| BHT | | 0.05 |
| PEG 90 M | | 0.1 |
| Eau | | 89.35 |

**Tableau 2 - Activateur biphasique**

| **Ingrédients** | **Quantité (% p/p)** |
|---|---|
| Huile de brocoli | 75 |
| Ethanol | 20 |
| Glycérine | 2,5 |
| Eau | 2,5 |

### Exemple 2 - Test de lissage

### 2.1 - Procédé de lissage

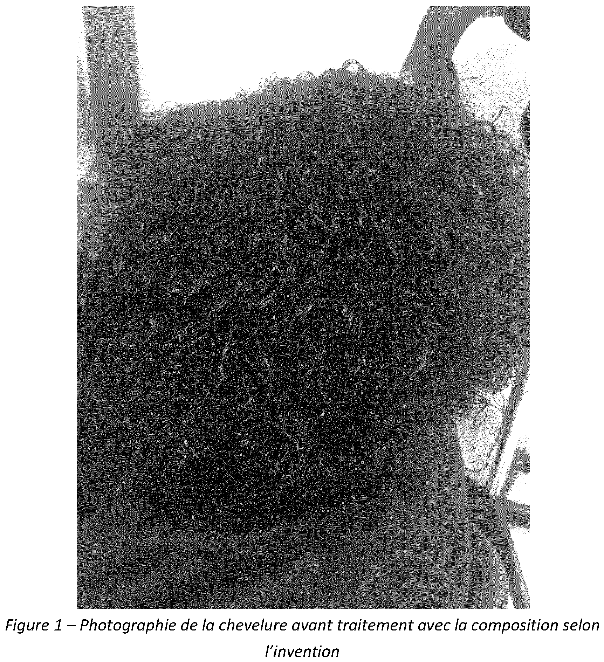

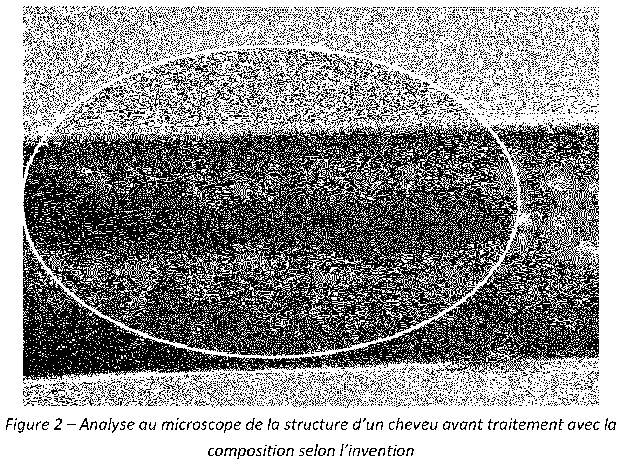

Le kit décrit dans l'exemple 1 précédent est utilisé en vue de procéder au lissage d'une chevelure de type afro, très fragilisée par d'anciens défrisages à l'acide thio jusqu'à obtenir une casse importante sur toute la partie du plateau empêchant toute utilisation de produit à base de réducteur fort (Figure 1). Une analyse au microscope d'un cheveu avant traitement révèle d'ailleurs une nette corrosion de la structure interne (Figure 2).

L'application de la composition selon l'invention est effectuée selon le protocole suivant.

### Etape 1 : Préparation des cheveux

On effectue un double shampooing à l'aide d'un shampooing préparateur.

### Etape 2 : Préparation de la composition selon l'invention

On mélange la composition de lissage et l'activateur bi-phasique de l'exemple 1 dans un rapport de dilution composition de lissage/activateur bi-phasique de 60/40.

### Etape 3 : Application de la composition selon l'invention

On démêle et on pré-sèche la chevelure à 70 % à l'aide d'un séchoir.

La composition selon l'invention est appliquée mèches par mèches de la racine à la pointe. Chaque mèche est malaxée et peignée afin de bien répartir le produit.

La chevelure est ensuite recouverte d'une cellophane et on laisse agir la composition durant 30 minutes.

### Etape 4 : Séchage

Les cheveux sont ensuite séchés à l'air chaud avec un séchoir, en orientant le souffle de l'air de la racine à la pointe pour descendre la cuticule. Des mèches de 3 cm de large sont sélectionnées et maintenues perpendiculaires au cuir chevelu afin d'être séchées à 100%.

### Etape 5 : Rinçage

On laisse ensuite le cheveu refroidir et on rince les cheveux à l'eau. Puis on applique une composition « masque » classique et on laisse agir 5 minutes avant de rincer à nouveau les cheveux à l'eau.

### Etape 6 : Lissage

Les cheveux sont ensuite coiffés à la main avec un séchoir.

### 2.2 - Résultats obtenus

On remarque que lors du lissage, les cheveux se lissent directement et se mettent en place sans que l'on ait besoin d'utiliser un fer à lisser.

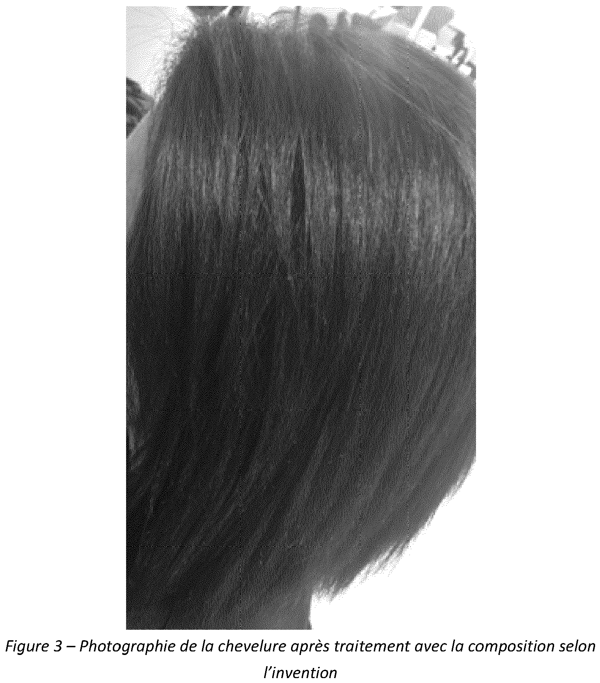

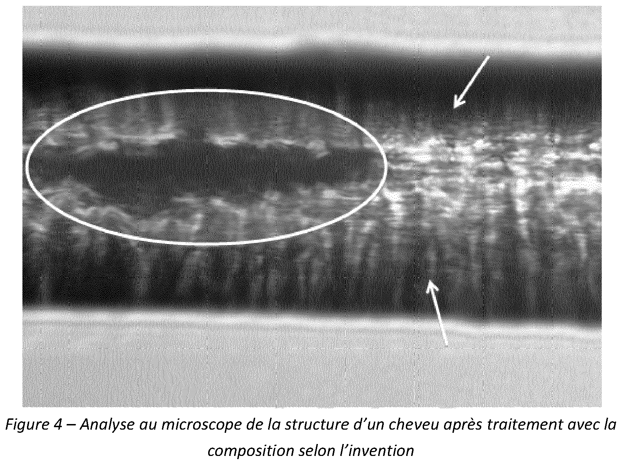

On obtient ainsi un cheveu lisse simplement au contact de la chaleur du séchoir, sans avoir recours à l'effet de traction ou de chaleur intense du fer à lisser. Le volume est maîtrisé et on note un effet de reconstruction important de cette fibre qui était préalablement sensibilisée (Figure 3). L'analyse au microscope d'un cheveu après traitement révèle d'ailleurs la présence du lissage sur toute la surface de la fibre ainsi qu'une amélioration de la structure interne en comparaison du cheveu avant traitement (Figure 4).

Une brillance intense est observée et la chevelure a retrouvé une fluidité et une légèreté importante.

En outre, les effets observés sont durables (environ trois mois).

### Exemple 3 - Kit selon l'invention

Un kit contenant :
- une composition de lissage se présentant sous la forme d'une crème lissante comprenant un acide aminé protéinogène, une protéine, un alcool et une huile végétale ;
- et un masque « top coat » comprenant une protéine et une résine hydrophobe ; dont les compositions sont rapportées respectivement dans les Tableaux 3 et 4 suivants est préparé.

**Tableau 3 - Composition de lissage**

| **Ingrédients** | **Quantité (% p/p)** |
|---|---|
| Protéine de blé hydrolysée | 4 |
| Kératine hydrolysée | 4 |
| Huile de brocoli | 18 |
| Glycerine | 3.7 |
| Arginine | 0.8 |
| Cysteine | 1 |
| Acide glutamique | 0.4 |
| Maltose | 1 |
| Alcool | 3.8 |
| Caprylic capric tryglyceride | 5 |
| Tocopherol | 0.2 |
| Jus d'aloe vera | 20 |
| Huile de coco | 8 |
| Glycéryl stéarate citrate | 2 |
| Hydroxypropyl starch phosphate | 3 |
| Cetearyl alcohol | 2 |
| Sodium stearoyl lactilate | 1 |
| Ceteareth-20 | 3 |
| Phénoxyethanol | 0.5 |
| Acide dehydroacetic, alcool benzylique | 0.4 |
| Parfum | 0.5 |
| Eau | 17.7 |

**Tableau 4 - Masque « top coat »**

| **Ingrédients** | **Quantité (% p/p)** |
|---|---|
| Eau | 77.6 |
| Cetearyl alcohol | 3.5 |
| Cetrimonium chloride | 6.6 |
| Glycerine | 0.5 |
| Huile de babassu | 0.5 |
| Behentrimonium methosulfate, cetearyl alcohol | 2 |
| Huile de germe de blé | 0.5 |
| Polyquaternium-7 | 0.5 |
| Beurre de karité | 2 |
| Cetrimonium chloride, amodimethicone, trideceth-12 | 1 |
| Acide Lactique | 0.05 |
| Phenoxyethanol | 0.4 |
| Kératine hydrolysée | 1 |
| Triacontanyl PVP (résine waterproof) | 1 |
| Collagène hydrolysé | 0.4 |
| Aminopropylphenyltrimethicone | 0.4 |
| Guar hydroxypropyltrimonium chloride | 0.1 |
| PEG-90M | 0.1 |
| Elastine hydrolysée | 0.8 |
| Panthenol | 0.5 |
| BHT | 0.05 |
| Parfum | 0.5 |

### Exemple 4 - Test de lissage

### 4.1 - Procédé de lissage

Le kit décrit dans l'exemple 3 précédent est utilisé en vue de procéder au lissage d'une chevelure crépue type afro, récalcitrante et indomptable lorsqu'elle entre en contact avec l'humidité.

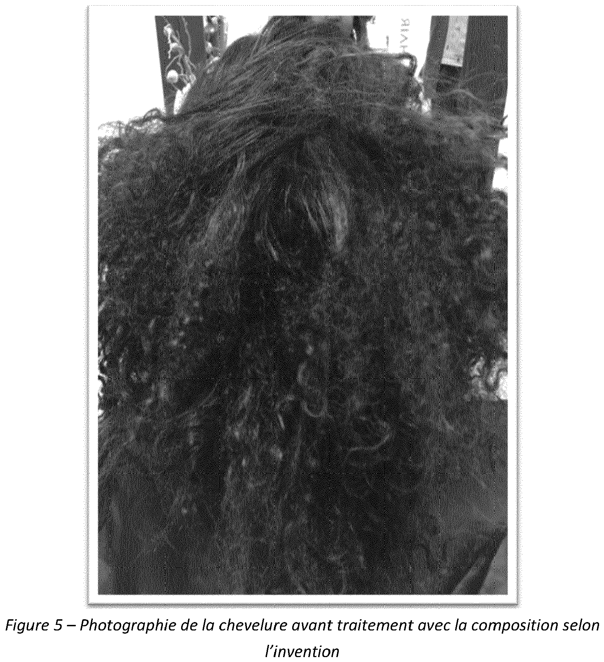

Une photo faite avant traitement montre la chevelure dans son état naturel (Figure 5).

L'application de la composition selon l'invention est effectuée selon le protocole suivant.

### Etape 1 : Application de la crème lissante directement sur cheveux secs

La composition selon l'invention est appliquée mèches par mèches de la racine à la pointe. Chaque mèche est malaxée et peignée afin de bien répartir le produit.

On recouvre ensuite la chevelure d'une cellophane et on laisse agir la composition durant 30 minutes.

### Etape 2 : Séchage

Les cheveux sont ensuite chauffés à l'air chaud du séchoir, en orientant le souffle de l'air de la racine à la pointe pour descendre la cuticule. Des mèches de 3 cm de large sont sélectionnées et maintenues perpendiculaires au cuir chevelu afin d'être chauffées sur toute leur longueur.

### Etape 3 : Rinçage

On rince la totalité de la chevelure et on applique un shampooing classique sans Sodium Laureth Sulfate (SLS).

On rince le shampooing et on applique la composition masque « top coat », puis on laisse pauser 5 minutes et on rince.

### Etape 4 ; Lissage

Les cheveux sont ensuite coiffés à la main avec un séchoir.

### 4.2- Résultats obtenus

On remarque que lors du lissage, les cheveux se lissent directement et se mettent en place sans que l'on ait besoin d'utiliser un fer à lisser.

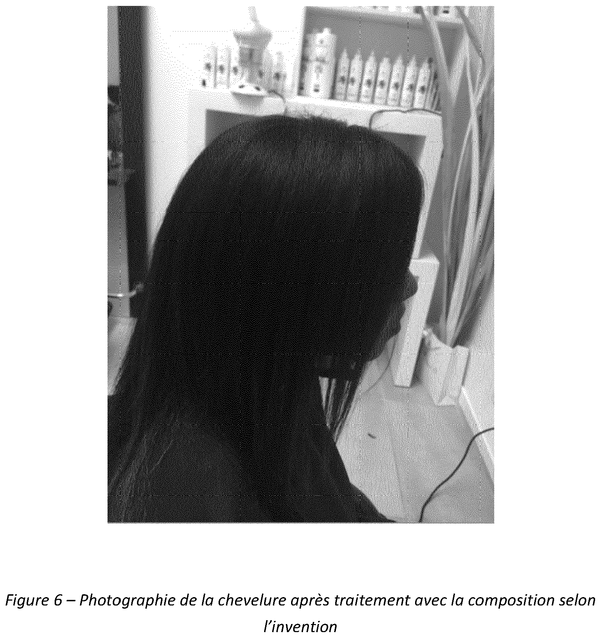

On obtient ainsi un cheveu lisse simplement au contact de la chaleur du séchoir, sans avoir recours à l'effet de traction ou de chaleur intense du fer à lisser (Figure 6).

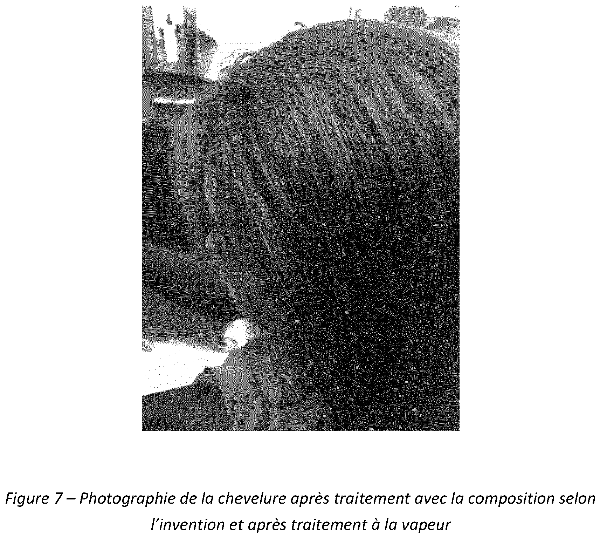

La fibre est maîtrisée, y compris après avoir été soumise à pendant une durée de 15 minutes à un traitement vapeur à un taux d'humidité de 91% (Figure 7).

En outre, les effets observés sont durables (trois mois environ).

### Exemple 5 - Test comparatif

Un kit contenant :
- une composition de lissage se présentant sous la forme d'une crème lissante comprenant uniquement un acide aminé protéinogène et un alcool (i.e. sans protéine et sans huile végétale) ;
- et un masque « top coat » comprenant une protéine ;
dont les compositions sont rapportées respectivement dans les Tableaux 5 et 6 suivants est préparé.

**Tableau 5- Composition de lissage**

| **Ingrédients** | **Quantité (% p/p)** |
|---|---|
| Eau | 51.9 |
| Glycérine | 2 |
| Arginine | 1 |
| Cysteine | 1.8 |
| Acide glutamique | 0.4 |
| Alcool | 5 |
| Caprylic capric tryglyceride | 5 |
| Tocopherol | 0.5 |
| Jus d'aloe vera | 20 |
| Glycéryl stéarate citrate | 2 |
| Hydroxypropyl starch phosphate | 3 |
| Cetearyl alcohol | 2 |
| Sodium stearoyl lactilate | 1 |
| Ceteareth-20 | 3 |
| Phénoxyethanol | 0.5 |
| Acide dehydroacetic, alcool benzylique | 0.4 |
| Parfum | 0.5 |

**Tableau 6 - Masque « top coat »**

| **Ingrédients** | **Quantité (% w/w)** |
|---|---|
| Eau | 80.6 |
| Cetearyl alcohol | 3.5 |
| Cetrimonium chloride | 6.6 |
| Glycerine | 0.5 |
| Behentrimonium methosulfate, cetearyl alcohol | 2 |
| Polyquaternium-7 | 0.5 |
| Beurre de karité | 2 |
| Cetrimonium chloride, amodimethicone, trideceth-12 | 1 |
| Acide Lactique | 0.05 |
| Phenoxyethanol | 0.4 |
| Collagène hydrolysé | 0.4 |
| Aminopropylphenyltrimethicone | 0.4 |
| Guar hydroxypropyltrimonium chloride | 0.1 |
| PEG-90M | 0.1 |
| Elastine hydrolysée | 0.8 |
| Panthenol | 0.5 |
| BHT | 0.05 |
| Parfum | 0.5 |

Un test de lissage a par la suite été effectué avec cette composition selon un protocole identique à celui décrit dans l'exemple 4.

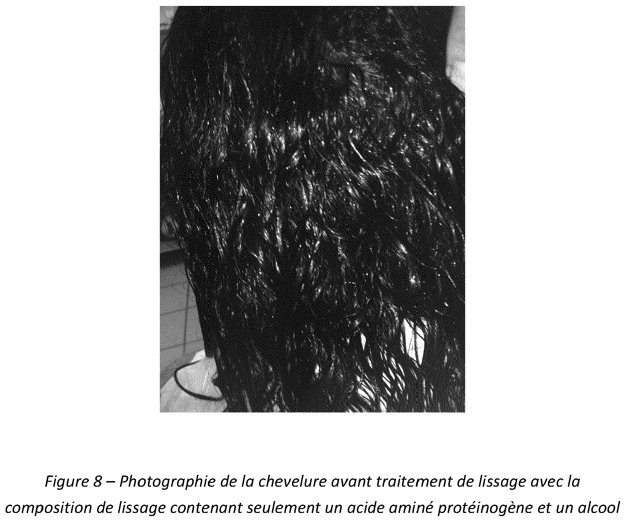

Une photo faite avant traitement montre la chevelure dans son état naturel (Figure 8).

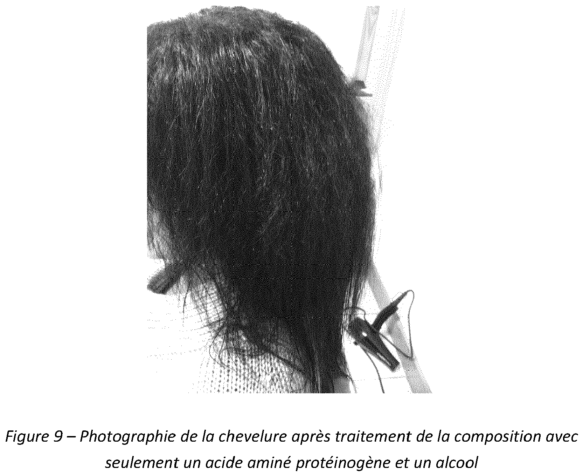

Après traitement, on observe une chevelure terne et déshydratée. En outre, au contact de la chaleur du séchoir, la chevelure ne se lisse pas mais gonfle et prend une apparence très mousseuse. Malgré une tentative de reprise à la brosse, le résultat est très insuffisant (Figure 9).

## Revendications

1. Composition pour le lissage des cheveux comprenant :
- un acide aminé protéinogène ou l'un de ses sels, d'acide ou de base, organiques ou minéraux ;
- une protéine ;
- un alcool ; et
- une huile végétale.

2. Composition selon la revendication 1, **caractérisée en ce que** l'acide aminé est choisi parmi l'acide aspartique, l'acide glutamique, l'asparagine, la carnitine, la cystéine, la glutamine, l'histidine, la leucine, l'isoleucine, la méthionine, la N-phenylalanine, la proline, l'hydroxyproline, la threonine, le tryptophan, la tyrosine, la valine, la glycine, l'alanine, la sérine, la béta alanine, la taurine, la lysine et l'arginine ou l'un de leurs sels, d'acide ou de base, organiques ou minéraux.

3. Composition selon la revendication 1 ou 2, **caractérisée en ce que** la protéine est choisie parmi la créatine éventuellement hydrolysée, la kératine éventuellement hydrolysée, la protéine de blé éventuellement hydrolysée, la protéine de soie éventuellement hydrolysée, la protéine de soja éventuellement hydrolysée, le collagène éventuellement hydrolysé, l'élastine éventuellement hydrolysée, la protéine de pois éventuellement hydrolysée, la protéine de riz éventuellement hydrolysée, la protéine de conchioline éventuellement hydrolysée et la protéine de lactosérum éventuellement hydrolysée.

4. Composition selon la revendication 3, **caractérisée en ce que** la protéine est choisie parmi la créatine, la kératine, la protéine de blé éventuellement hydrolysée, la protéine de soie éventuellement hydrolysée et la protéine de soja éventuellement hydrolysée.

5. Composition selon l'une des revendications 1 à 4, **caractérisée en ce que** l'alcool est choisi parmi le butanol, l'isopropanol et l'éthanol.

6. Composition selon la revendication 5, **caractérisée en ce que** l'alcool est l'éthanol.

7. Composition selon l'une des revendications 1 à 6, **caractérisée en ce que** l'huile végétale est choisie parmi l'huile d'amande douce, l'huile d'olive, l'huile d'argan, l'huile d'Haarlem, l'huile de Babassu, l'huile de brocoli, l'huile d'Avocat, l'huile de Lin, l'huile d'abricot, l'huile de concombre, l'huile de nigelle, l'huile de noix du brésil, l'huile de pépins de raisin, l'oléine de karité, l'huile de sésame et l'huile de tomate.

8. Composition selon la revendication 7, **caractérisée en ce que** l'huile végétale est l'huile de brocoli.

9. Kit utile pour le lissage des cheveux comprenant :
- d'une part une composition contenant l'acide aminé selon la revendication 1 ou 2, et la protéine selon l'une des revendications 1, 3 ou 4 ; et
- d'autre part une composition contenant l'alcool selon l'une des revendications 1, 5 ou 6, et l'huile végétale selon l'une des revendications 1, 7 ou 8.

10. Kit selon la revendication 9, **caractérisé en ce qu'**il contient de 50% à 70% de la composition comprenant l'acide aminé et la protéine et de 30% à 50% de la composition comprenant l'alcool et l'huile végétale.

11. Kit selon la revendication 9 ou 10, caratérisé en ce que la composition contenant l'acide aminé et la protéine se présente sous la forme de crème, de gel, d'émulsion huile dans eau ou eau dans huile, de lotion capillaire ou de shampooing.

12. Kit selon l'une quelconque des revendications 9 à 11, **caractérisé en ce que** la composition contenant l'alcool et l'huile végétale se présente sous la forme de crème, de gel, d'émulsion huile dans eau ou eau dans huile, de lotion bi-phasique ou de shampooing.

13. Procédé de lissage des cheveux comprenant les étapes suivantes :
- lorsque la composition se présente sous forme d'un kit selon l'une des revendications 9 à 12, mélanger la composition comprenant l'acide aminé et la protéine et la composition comprenant l'alcool et l'huile végétale ;
- appliquer le produit obtenu mèches par mèches de la racine à la pointe
- laisser agir le produit pendant une durée pouvant varier de 15 minutes à 45 minutes ;
- sécher les cheveux ;
- rincer les cheveux à l'eau ; et
- procéder au coiffage avec un séchoir.

## Patentansprüche

1. Zusammensetzung zum Glätten von Haaren, die umfasst:
- eine proteinogene Aminosäure oder eins ihrer Salze, sauer oder basisch, organisch oder mineralisch;
- ein Protein;
- einen Alkohol; und
- ein Pflanzenöl.

2. Zusammensetzung nach Anspruch 1, **dadurch gekennzeichnet, dass** die Aminosäure aus der Asparaginsäure, der Glutaminsäure, Asparagin, Carnitin, Cystein, Glutamin, Histidin, Leucin, Isoleucin, Methionin, N-Phenylalanin, Prolin, Hydroxyprolin, Threonin, Tryptophan, Tyrosin, Valin, Glycin, Alanin, Serin, Beta-Alanin, Taurin, Lysin und Arginin oder einem ihrer Salze, sauer oder basisch, organisch oder mineralisch, ausgewählt ist.

3. Zusammensetzung nach Anspruch 1 oder 2, **dadurch gekennzeichnet, dass** das Protein aus dem gegebenenfalls hydrolysierten Creatin, dem gegebenenfalls hydrolysierten Keratin, dem gegebenenfalls hydrolysierten Weizenprotein, dem gegebenenfalls hydrolysierten Seidenprotein, dem gegebenenfalls hydrolysierten Sojaprotein, dem gegebenenfalls hydrolysierten Collagen, dem gegebenenfalls hydrolysierten Elastin, dem gegebenenfalls hydrolysierten Erbsenprotein, dem gegebenenfalls hydrolysierten Reisprotein, dem gegebenenfalls hydrolysierten Conchiolinprotein und dem gegebenenfalls hydrolysierten Molkeprotein ausgewählt ist.

4. Zusammensetzung nach Anspruch 3, **dadurch gekennzeichnet, dass** das Protein aus dem Creatin, dem Keratin, dem gegebenenfalls hydrolysierten Weizenprotein, dem gegebenenfalls hydrolysierten Seidenprotein und dem gegebenenfalls hydrolysierten Sojaprotein ausgewählt ist.

5. Zusammensetzung nach einem der Ansprüche 1 bis 4, **dadurch gekennzeichnet, dass** der Alkohol aus Butanol, Isopropanol und Ethanol ausgewählt ist.

6. Zusammensetzung nach Anspruch 5, **dadurch gekennzeichnet, dass** der Alkohol Ethanol ist.

7. Zusammensetzung nach einem der Ansprüche 1 bis 6, **dadurch gekennzeichnet, dass** das Pflanzenöl aus dem Süßmandelöl, dem Olivenöl, dem Arganöl, dem Haarlem-Öl, dem Babassu-Öl, dem Brokkoliöl, dem Avocadoöl, dem Leinöl, dem Aprikosenöl, dem Gurkenöl, dem Schwarzkümmelöl, dem Paranussöl, dem Traubenkernöl, dem Shea-Olein, dem Sesamöl und dem Tomatenöl ausgewählt ist.

8. Zusammensetzung nach Anspruch 7, **dadurch gekennzeichnet, dass** das Pflanzenöl das Brokkoliöl ist.

9. Kit zum Glätten der Haare, das umfasst:
- zum einen eine Zusammensetzung, die die Aminosäure nach Anspruch 1 oder 2 und das Protein nach einem der Ansprüche 1, 3 oder 4 enthält; und
- zum anderen eine Zusammensetzung, die den Alkohol nach einem der Ansprüche 1, 5 oder 6 und das Pflanzenöl nach einem der Ansprüche 1, 7 oder 8 enthält.

10. Kit nach Anspruch 9, **dadurch gekennzeichnet, dass** es 50 % bis 70 % der Zusammensetzung, die die Aminosäure und das Protein umfasst, und 30 % bis 50 % der Zusammensetzung, die den Alkohol und das Pflanzenöl umfasst, enthält.

11. Kit nach Anspruch 9 oder 10, **dadurch gekennzeichnet, dass** die Zusammensetzung, die die Aminosäure und das Protein enthält, in Form von Creme, Gel, Öl-in-Wasser- oder Wasser-in-Öl-Emulsion, Haarlotion oder Shampoon vorliegt.

12. Kit nach einem der Ansprüche 9 bis 11, **dadurch gekennzeichnet, dass** die Zusammensetzung, die den Alkohol und das Pflanzenöl enthält, in Form von Creme, Gel, Öl-in-Wasser- oder Wasser-in-Öl-Emulsion, Zweiphasenlotion oder Shampoon vorliegt.

13. Verfahren zum Glätten von Haaren, das die folgenden Schritte umfasst:
- wenn die Zusammensetzung in Form eines Kits nach einem der Ansprüche 9 bis 12 vorliegt, Mischen der Zusammensetzung, die die Aminosäure und das Protein umfasst, und der Zusammensetzung, die den Alkohol und das Pflanzenöl umfasst;
- Auftragen des erhaltenen Produkts auf jede einzelne Strähne von der Wurzel bis zur Spitze;
- Wirken lassen des Produkts während einer Dauer, die von 15 Minuten bis 45 Minuten schwanken kann;
- Trocknen der Haare;
- Abspülen der Haare mit Wasser; und
- Frisieren mit einem Fön.

## Claims

1. A composition for straightening hair comprising:
- a proteinogenic amino acid or one of its acid or base, organic or mineral salts;
- a protein;
- an alcohol; and
- a vegetable oil.

2. The composition according to claim 1, **characterized in that** the amino acid is selected from aspartic acid, glutamic acid, asparagine, carnitine, cysteine, glutamine, histidine, leucine, isoleucine, methionine, N-phenylalanine, proline, hydroxyproline, threonine, tryptophan, tyrosine, valine, glycine, alanine, serine, beta alanine, taurine, lysine and arginine or one of their acid or base, organic or mineral salts.

3. The composition according to claim 1 or 2, **characterized in that** the protein is selected from possibly hydrolyzed creatine, possibly hydrolyzed keratin, possibly hydrolyzed wheat protein, possibly hydrolyzed silk protein, possibly hydrolyzed soy protein, possibly hydrolyzed collagen, possibly hydrolyzed elastin, possibly hydrolyzed pea protein, possibly hydrolyzed rice protein, possibly hydrolyzed conchiolin protein and possibly hydrolyzed whey protein.

4. The composition according to claim 3, **characterized in that** the protein is selected from creatine, keratin, possibly hydrolyzed wheat protein, possibly hydrolyzed silk protein and possibly hydrolyzed soy protein.

5. The composition according to any of claims 1 to 4, **characterized in that** the alcohol is selected from butanol, isopropanol and ethanol.

6. The composition according to claim 5, **characterized in that** the alcohol is ethanol.

7. The composition according to any of claims 1 to 6, **characterized in that** the vegetable oil is selected from sweet almond oil, olive oil, argan oil, Haarlem oil, Babassu oil, broccoli seed oil, avocado oil, linseed oil, apricot oil, cucumber oil, nigella oil, brazil nut oil, grape seed oil, shea olein, sesame oil and tomato oil.

8. The composition according to claim 7, **characterized in that** the vegetable oil is broccoli seed oil.

9. A kit useful for straightening hair comprising:
- on the one hand a composition containing the amino acid according to claim 1 or 2, and the protein according to any of claims 1, 3 or 4; and
- on the other hand, a composition containing the alcohol according to any of claims 1, 5 or 6, and the vegetable oil according to any of claims 1, 7 or 8.

10. The kit according to claim 9, **characterized in that** it contains from 50% to 70% of the composition comprising the amino acid and the protein and from 30% to 50% of the composition comprising the alcohol and the vegetable oil.

11. The kit according to claim 9 or 10, **characterized in that** the composition containing the amino acid and the protein is in the form of a cream, gel, oil-in-water or water-in-oil emulsion, hair lotion or shampoo.

12. The kit according to any one of claims 9 to 11, **characterized in that** the composition containing the alcohol and the vegetable oil is in the form of a cream, gel, oil-in-water or water-in-oil emulsion, bi-phasic lotion or shampoo.

13. A method for straightening hair comprising the following steps:
- when the composition is in the form of a kit according to any of claims 9 to 12, mix the composition comprising the amino acid and the protein and the composition comprising the alcohol and the vegetable oil;
- apply the obtained product strands by strands from the root to the tip;
- leave the product to act for a period which may vary from 15 minutes to 45 minutes;
- dry the hair;
- rinse the hair with water; and
- proceed with styling with a hair dryer.
